# FASCICULE DE BREVET EUROPEEN

(11) **EP 4 003 588 B1**
(45) Date de publication et mention de la délivrance du brevet: **06.09.2023**
(21) Numéro de dépôt: 20739705.0
(22) Date de dépôt: 16.07.2020
(51) Int. Cl.: B01J 21/04, B01J 23/89, B01J 37/10, B01J 35/10, B01J 37/02, B01J 23/755, B01J 37/08, B01J 37/16, C07C 5/09, C07C 5/11, C10G 45/36, C07C 5/10

(54) **PROCEDE DE PREPARATION D'UN CATALYSEUR COMPRENANT UNE PHASE ACTIVE DE NICKEL REPARTIE EN CROUTE**
VERFAHREN ZUR HERSTELLUNG EINES KATALYSATORS MIT EINER IN EINER SCHALE VERTEILTEN AKTIVEN NICKELPHASE
PREPARATION PROCESS OF A CATALYST COMPRISING AN ACTIVE NICKEL PHASE DISTRIBUTED IN A SHELL

(30) Priorité: 31.07.2019 FR 1908719
(43) Date de publication de la demande: 01.06.2022
(73) Titulaire: IFP Energies nouvelles, 92852 Rueil-Malmaison Cedex (FR)
(72) Inventeur: DUBREUIL, Anne-Claire, 92852 RUEIL-MALMAISON CEDEX (FR); COUPARD, Vincent, 92852 RUEIL-MALMAISON CEDEX (FR); BOUALLEG, Malika, 92852 RUEIL-MALMAISON CEDEX (FR); CHOLLET, Carine, 92852 RUEIL-MALMAISON CEDEX (FR); JOUVE, Alexandre, 92852 RUEIL-MALMAISON CEDEX (FR)
(74) Mandataire: IFP Energies nouvelles
(86) Numéro de dépôt international: PCT/EP2020/070082
(87) Numéro de publication internationale: WO 2021/018604

(56) Documents cités:
- FR-A1- 2 927 267
- FR-A1- 3 064 500
- FR-A1- 3 076 746
- US-A1- 2012 065 442

## Description

### Domaine technique

La présente invention concerne un procédé de préparation d'un catalyseur métallique supporté à base de nickel destiné particulièrement à l'hydrogénation des hydrocarbures insaturés, et plus particulièrement, d'hydrogénation sélective de composés polyinsaturés ou d'hydrogénation des aromatiques.

### Etat de la technique

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés styréniques ou indéniques. Ces composés polyinsaturés sont très réactifs et conduisent à des réactions parasites dans les unités de polymérisation. Il est donc nécessaire de les éliminer avant de valoriser ces coupes. L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants.

Les catalyseurs d'hydrogénation sélective sont généralement à base de métaux du groupe VIII du tableau périodique, de préférence le palladium ou le nickel. Le métal se présente sous la forme de particules métalliques déposées sur un support. La teneur en métal, la taille des particules de métal et la répartition de la phase active dans le support font partie des critères qui ont une importance sur l'activité et la sélectivité des catalyseurs.

La répartition macroscopique des particules métalliques dans le support constitue un critère important, principalement dans le cadre de réactions rapides et consécutives telles que les hydrogénations sélectives. Il est généralement souhaitable que ces éléments se situent dans une croûte à la périphérie du support afin d'éviter les problèmes de transfert de matière intragranulaire pouvant conduire à des défauts d'activité et une perte de sélectivité. De tels catalyseurs sont aussi appelé catalyseurs "eggshell" selon la terminologie anglo-saxonne. De tels catalyseurs sont largement connus dans le cas des catalyseurs d'hydrogénation sélective à base de palladium. En effet, grâce à la faible teneur en palladium (généralement inférieure à 1 % en poids (1 % pds) de palladium par rapport au catalyseur) et des procédés de préparation adaptés, une croûte fine de palladium à la périphérie des grains de support peut être obtenue (FR2922784, US2010/217052).

Il est souvent proposé de substituer le palladium par le nickel, métal moins actif que le palladium qu'il est donc nécessaire de disposer en plus grande quantité dans le catalyseur. Ainsi, les catalyseurs à base de nickel ont généralement une teneur en métal entre 5 et 50 % pds de nickel par rapport au catalyseur. Dans ces catalyseurs, le nickel est généralement réparti de façon homogène au sein du support. Une des voies d'amélioration possible de ces catalyseurs en termes d'activité et de sélectivité est de contrôler la répartition du nickel au sein du support en déposant le nickel de façon plus concentrée sur une croûte, à la périphérie du support. De tels catalyseurs sont connus de l'état de l'art.

Le document US 4 519 951 décrit un catalyseur de type « eggshell » avec du nickel sur un support poreux ayant un volume poreux des pores dont la taille est inférieure à 11,7 nm d'au moins 0,2 ml/g et un volume poreux des pores dont la taille est supérieure à 11,7 nm d'au moins 0,1 ml/g. Plus de 50 % du nickel se trouve dans une croûte dont l'épaisseur est égale à 0,15 fois le rayon du support. Ce catalyseur est utilisé pour l'hydrogénation de matières grasses.

Le document CN101890351 décrit un catalyseur supporté de nickel dans lequel plus de 90 % du nickel se trouve dans une croûte de 700 µm d'épaisseur. Le catalyseur est préparé en utilisant une solution ammoniacale pour dissoudre le sel de nickel. Ces catalyseurs sont utilisés dans une application d'hydrogénation sélective.

Le document US2012/0065442 décrit un catalyseur supporté de nickel dans lequel la distribution de la taille des cristallites de nickel est bimodale avec 30 à 70% des cristallites de nickel ayant une taille moyenne (diamètre) de 1,0 à 2,5 nm, les cristallites de nickel restants ayant une taille moyenne (diamètre) de 3,0 à 4,5 nm. Le nickel est reparti à la fois sur une croûte d'une épaisseur de 3 à 15 % du diamètre et à coeur, le ratio de concentration en nickel entre la croûte et le coeur étant compris entre 3,0 : 1 et 1,3 : 1. Au moins 75 % du volume poreux se trouve dans des pores ayant une taille de plus de 5,0 nm.

La demande FR3064500 divulgue un catalyseur comprenant au moins un métal du groupe VIII et un support comprenant au moins un oxyde réfractaire sélectionné dans le groupe constitué par la silice, l'alumine et la silice-alumine, ledit catalyseur étant obtenu par un procédé de préparation comprenant au moins les étapes suivantes :
a) on met en contact ledit support poreux avec au moins une solution contenant au moins un compose organique comprenant au moins une fonction acide carboxylique ;
b) on met en contact ledit support poreux avec au moins une solution contenant au moins un précurseur de métal du groupe VIII ;
   les étapes a) et b) étant réalisées séparément, dans un ordre indiffèrent, ou simultanément;
c) on sèche le support imprégné a une température comprise entre 15°Cet inférieure ou égale à 250°C ;
d) on calcine le support sèche issu de l'étape c) a une température supérieure à 250°C mais inférieure à 900°C.

La demande FR2927267 divulgue un procédé de préparation d'un catalyseur d'hydrogénation sélective comprenant au moins les étapes suivantes :
a) on approvisionne un gel d'alumine de type hydrargilite ;
b) on met en forme le gel de l'étape a) par calcination flash, broyage, lavage et granulation ;
c) on soumet le gel d'alumine mis en forme obtenu à l'issue de l'étape b) à un traitement thermique comprenant au moins une étape de traitement hydrothermal dans un autoclave en présence d'une solution acide à une température de 200°C puis on réalise une étape de calcination à une température de 950°C pour obtenir un support d'alumine ;
d) on imprègne le support d'alumine obtenu à l'issue de l'étape c) avec une solution aqueuse de nitrate de nickel pour obtenir un précurseur de catalyseur ;
e) on sèche le précurseur de catalyseur obtenu à l'issue de l'étape d) à une température de 120°C ;
e') on calcine le précurseur de catalyseur obtenu à l'issue de l'étape e) à une température de 450°C pour obtenir un catalyseur ;
f) on met en contact le catalyseur obtenu à l'issue de l'étape e') avec au moins une solution contenant de l'acide formique ;
g) on réalise un traitement thermal du précurseur de catalyseur obtenu à l'issue de l'étape f) à une température de 150°C.

La demande FR3076746 divulgue un procédé de préparation d'un catalyseur comprenant une matrice oxyde et une phase active comprenant du nickel comprenant les étapes suivantes :
- on prépare un oxyde poreux aluminique calciné ;
- on malaxe l'oxyde poreux aluminique calciné obtenu avec au moins une solution comprenant au moins un précurseur de nickel pour obtenir une pâte, à une concentration en nickel voulue pour obtenir sur le catalyseur séché ou calciné une teneur comprise entre 10 et 35 % poids de nickel par rapport au poids total du catalyseur ;
- on met en forme la pâte obtenue ;
- on sèche la pâte mise en forme obtenue à une température inférieure à 250°C pour obtenir un précurseur de catalyseur séché ;
- on imprègne le précurseur de catalyseur séché obtenu avec au moins une solution comprenant au moins un précurseur de nickel, pour obtenir un précurseur de catalyseur imprégné ;
- on sèche le précurseur de catalyseur imprégné obtenu à une température inférieure à 250°C pour obtenir un catalyseur séché.

### Objets de l'invention

De manière surprenante, la Demanderesse a découvert qu'en appliquant un traitement hydrothermal spécifique après l'ajout d'un additif organique particulier sur un catalyseur à base de nickel comprenant un support d'alumine obtenu selon une méthode bien particulière, on obtient un catalyseur dans lequel au moins une partie du nickel est répartie sur une croûte à la périphérie du support, l'autre partie du nickel étant répartie au coeur du catalyseur. Sans vouloir être lié par une quelconque théorie, le traitement hydrothermal réalisé après l'étape de mise en contact d'un additif organique spécifique sur le catalyseur à base de nickel sur un support d'alumine particulier, ayant subi un traitement hydrothermal en présence d'une solution acide, semble faire migrer au moins en partie le nickel de l'intérieur du support à la périphérie du support formant ainsi une croûte de nickel. La présente invention concerne ainsi un nouveau type de catalyseur qui, de par son procédé de préparation spécifique, permet l'obtention d'un catalyseur comprenant des performances au moins aussi bonnes, voire meilleures, en terme d'activité et de sélectivité dans le cadre des réactions d'hydrogénation sélective de composés polyinsaturés ou d'hydrogénation des aromatiques, tout en utilisant une quantité de phase de nickel inférieure à celle utilisée typiquement dans l'état de la technique, ce qui est due à une meilleure répartition de la phase active de nickel dans le support, rendant cette dernière plus accessible aux réactifs.

La présente invention a pour objet un procédé de préparation d'un catalyseur comprenant une phase active à base de nickel et un support d'alumine, ledit catalyseur comprenant entre 1 et 50 % poids de nickel élémentaire par rapport au poids total du catalyseur, ledit catalyseur étant caractérisé en ce que :
- le nickel est réparti à la fois sur une croûte en périphérie du support, et à coeur du support, l'épaisseur de ladite croûte étant comprise entre 2% et 15% du diamètre du catalyseur;
- le ratio de densité en nickel entre la croûte et le coeur est supérieur strictement à 3 ;
- ladite croûte comprend plus de 25% en poids élément nickel par rapport au poids total de nickel contenu dans le catalyseur,
- la taille des particules de nickel dans le catalyseur, mesurée sous forme oxyde, est comprise entre 7 et 25 nm.

Avantageusement, le ratio de densité en nickel entre la croûte et le coeur est supérieur ou égal à 3,5.

Avantageusement, ladite croûte comprend plus de 40% en poids élément nickel par rapport au poids total de nickel contenu dans le catalyseur.

Avantageusement, l'intervalle de transition entre le coeur et la croûte du catalyseur est compris entre 0,05% et 3% du diamètre du catalyseur.

Avantageusement, la taille des particules de nickel dans le catalyseur est comprise entre 8 et 23 nm.

Avantageusement, la teneur en soufre du support d'alumine est comprise entre 0,001% et 2% poids par rapport au poids total du support d'alumine, et la teneur en sodium dudit support d'alumine est comprise entre 0,001% et 2% poids par rapport au poids total dudit gel d'alumine.

Avantageusement, l'épaisseur de ladite croûte est comprise entre 2,5% et 12% du diamètre du catalyseur;
Avantageusement, le ratio de densité en nickel entre la croûte et le coeur est compris entre 3,8 et 15.

Un objet selon l'invention concerne un procédé de préparation d'un catalyseur selon l'invention, lequel procédé comprend les étapes suivantes :
a) on approvisionne un gel d'alumine ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on met en contact le support d'alumine obtenu à l'issue de l'étape c) avec au moins un précurseur de la phase active de nickel pour obtenir un précurseur de catalyseur,
e) on sèche le précurseur de catalyseur obtenu à l'issue de l'étape d) à une température inférieure à 250°C ;
f) on met en contact le précurseur de catalyseur séché obtenu à l'issue de l'étape e) avec au moins une solution contenant au moins un additif organique choisi parmi les aldéhydes renfermant 1 à 14 atomes de carbone par molécule, les cétones ou polycétones renfermant 3 à 18 atomes de carbone par molécule, les éthers et les esters renfermant 2 à 14 atomes de carbone par molécule, les alcools ou polyalcools renfermant 1 à 14 atomes de carbone par molécule et les acides carboxyliques ou polyacides carboxyliques renfermant 1 à 14 atomes de carbone par molécule, le ratio molaire entre l'additif organique et le nickel étant supérieur à 0,05 mol/mol ;
g) on réalise un traitement hydrothermal du précurseur de catalyseur obtenu à l'issue de l'étape f) à une température comprise entre 100 et 200°C sous flux gazeux comprenant entre 5 et 650 grammes d'eau par kg de gaz sec.

Avantageusement, le procédé comprend en outre une étape h) de séchage du précurseur de catalyseur obtenu à l'issue de l'étape g) à une température comprise entre 50 et 200°C sous flux gazeux comprenant une quantité d'eau inférieure strictement à 5 grammes d'eau par kg de gaz sec.

Avantageusement, le procédé comprend en outre une étape de calcination e1) du précurseur de catalyseur séché obtenu à l'issue de l'étape e), sous flux gazeux comprenant une quantité d'eau inférieure strictement à 150 grammes d'eau par kg de gaz sec à une température comprise entre 250°C et 1000°C.

Avantageusement, à l'étape f), l'additif organique est choisi parmi l'acide formique, le formaldéhyde, l'acide acétique, l'acide citrique, l'acide oxalique, l'acide glycolique, l'acide malonique, l'éthanol, le méthanol, le formiate d'éthyle, le formiate de méthyle, le paraldéhyde, l'acétaldéhyde, l'acide gamma-valérolactone, le glucose et le sorbitol, le trioxane. De préférence, l'additif organique est l'acide formique.

Le catalyseur préparé selon le procédé de l'invention peut être utilisé dans un procédé d'hydrogénation sélective de composés polyinsaturés contenant au moins 2 atomes de carbone par molécule contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur selon l'invention.

Le catalyseur préparé selon le procédé de l'invention peut être utilisé dans un procédé d'hydrogénation d'au moins un composé aromatique ou polyaromatique contenu dans une charge d'hydrocarbures ayant un point d'ébullition final inférieur ou égal à 650°C, ledit procédé étant réalisé en phase gazeuse ou en phase liquide, à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire (V.V.H.) comprise entre 0,05 et 50 h⁻¹, en présence d'un catalyseur selon l'invention.

### Description de la figure

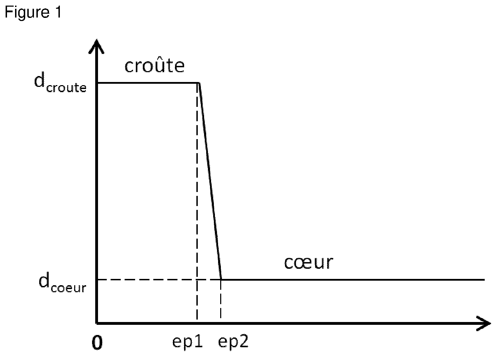

La figure 1 est un schéma représentant la répartition du nickel dans le catalyseur. L'axe des abscisses correspond à l'épaisseur du catalyseur, mesurée depuis le bord du catalyseur (en µm). L'axe des ordonnées correspond à la densité en nickel (en gramme de Ni / mm³). Le nickel est réparti à la fois sur une croûte en périphérie du support, d'épaisseur ep1, et à coeur du support. La densité en nickel sur la croûte d_{croute} est supérieure à la densité en nickel au coeur du support d_{coeur}. L'intervalle de transition entre le coeur et la croûte du catalyseur a une épaisseur notée ep2-ep1.

### Description détaillée de l'invention

### 1. Définitions

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC. Dans la présente description, on entend, selon la convention IUPAC, par micropores les pores dont le diamètre est inférieur à 2 nm, c'est à dire 0,002 µm; par mésopores les pores dont le diamètre est supérieur ou égal à 2 nm, c'est à dire 0,002 µm et inférieur ou égal à 50 nm, c'est à dire 0,05 µm et par macropores les pores dont le diamètre est supérieur à 50 nm, c'est à dire 0,05 µm.

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides. Cette méthode, bien connue de la personne du métier, est définie dans la publication de L. Sorbier et al. "Measurement of palladium crust thickness on catalyst by EPMA" Materials Science and Engineering 32 (2012). Elle permet d'établir le profil de répartition d'un élément donné, ici le Nickel, au sein du grain. Par ailleurs, la concentration en Ni est définie pour chaque mesure et donc pour chaque pas d'analyse. La densité de Ni au sein du grain est donc définie comme la concentration de Ni par mm³.

Le volume poreux total est mesuré par porosimétrie au mercure selon la norme ASTM D4284-92 avec un angle de mouillage de 140°, par exemple au moyen d'un appareil modèle Autopore III^{™} de la marque Microméritics^{™}.

La surface spécifique BET est mesurée par physisorption à l'azote selon la norme ASTM D3663-03, méthode décrite dans l'ouvrage Rouquerol F.; Rouquerol J.; Singh K. « Adsorption by Powders & Porous Solids: Principle, methodology and applications », Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On entend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the détermination of crystallite size», J. I. Langford and A. J. C. Wilson.

La teneur en nickel est mesurée par fluorescence X.

### 2. Catalyseur

L'invention porte sur un procédé de préparation d'un catalyseur comprenant, de préférence constitué de, une phase active à base de nickel, et un support d'alumine contenant avantageusement du soufre et du sodium, ledit catalyseur comprenant entre 1 et 50 % poids de nickel élémentaire par rapport au poids total du catalyseur, ledit catalyseur étant caractérisé en ce que :
- le nickel est réparti à la fois sur une croûte en périphérie du support, et à coeur du support, l'épaisseur de ladite croûte (appelée aussi ep1) étant comprise entre 2% et 15% du diamètre du catalyseur, de préférence entre 2,5% et 12% du diamètre du catalyseur, de façon encore plus préférée entre 3% et 10 % du diamètre du catalyseur et de façon encore plus préférée entre 3% et 7,5% du diamètre du catalyseur ;
- le ratio de densité en nickel entre la croûte et le coeur (appelé aussi ici d_{croute}/d_{coeur}) est supérieur strictement à 3, de préférence supérieur à 3,5 et de préférence compris entre 3,8 et 15 ;
- ladite croûte comprend plus de 25% en poids d'élément nickel par rapport au poids total d'élément nickel contenu dans le catalyseur, de préférence plus de 40% en poids, plus préférentiellement entre 45% et 90% en poids, et encore plus préférentiellement entre 60% et 90% en poids.

Avantageusement, l'intervalle de transition entre le coeur et la croûte du catalyseur (appelé aussi ici intervalle de transition coeur/croûte, ou ep2-ep1 d'après les notations de la figure 1), lié à la variation de la densité de nickel mesurée sur l'épaisseur du catalyseur depuis le bord du catalyseur jusqu'au centre du catalyseur, est très abrupte. De préférence, l'intervalle de transition coeur/croûte est compris entre 0,05 % et 3 % du diamètre du catalyseur, de préférence entre 0,5 % et 2,5 % du diamètre du catalyseur.

La teneur en nickel dans ledit catalyseur préparé selon l'invention est avantageusement comprise entre 1 et 50 % poids par rapport au poids total du catalyseur, plus préférentiellement entre 2 et 40 % poids et encore plus préférentiellement entre 3 et 35 % poids et encore plus préférentiellement 5 et 25% poids par rapport au poids total du catalyseur. Les valeurs « % poids » se basent sur la forme élémentaire du nickel.

Le catalyseur obtenu grâce au procédé selon l'invention peut être qualifié comme catalyseur « semi egg-shell » dans lequel la concentration du nickel est plus élevée en périphérie du support que dans le coeur du support, ladite concentration du nickel dans le coeur du support étant non nulle.

La surface spécifique du catalyseur est généralement comprise entre 10 m²/g et 200 m²/g, de préférence entre 25 m²/g et 110 m²/g, de façon plus préférée entre 40 m²/g et 100 m²/g. Le volume poreux total du catalyseur est généralement compris entre 0,1 et 1 ml/g, de préférence compris entre 0,2 ml/g et 0,8 ml/g, et de manière particulièrement préférée compris entre 0,3 ml/g et 0,7 ml/g.

La taille des particules de nickel, mesurée sous forme oxyde, dans le catalyseur est comprise entre 7 et 25 nm, de préférence entre 8 et 23 nm.

La phase active du catalyseur ne comprend pas de métal du groupe VIB. Elle ne comprend notamment pas de molybdène ou de tungstène.

Ledit catalyseur (et le support utilisé pour la préparation du catalyseur) est sous forme de grains ayant avantageusement un diamètre compris entre 0,5 et 10 mm. Les grains peuvent avoir toutes les formes connues de la personne du métier, par exemple la forme de billes (ayant de préférence un diamètre compris entre 1 et 8 mm), d'extrudés, de tablettes, de cylindres creux. De préférence, le catalyseur (et le support utilisé pour la préparation du catalyseur) sont sous forme d'extrudés de diamètre compris entre 0,5 et 10 mm, de préférence entre 0,8 et 3,2 mm et de manière très préférée entre 1,0 et 2,5 mm et de longueur comprise entre 0,5 et 20 mm. On entend par « diamètre» des extrudés le diamètre du cercle circonscrit à la section droite de ces extrudés. Le catalyseur peut être avantageusement présenté sous la forme d'extrudés cylindriques, multilobés, trilobés ou quadrilobés. De préférence sa forme sera trilobée ou quadrilobée. La forme des lobes pourra être ajustée selon toutes les méthodes connues de l'art antérieur.

### 3. Support

Les caractéristiques de l'alumine, mentionnées dans cette section, correspondent aux caractéristiques de l'alumine avant imprégnation de la phase active de nickel, i.e. le support d'alumine obtenu à l'issue de l'étape c) du procédé de préparation du catalyseur préparé selon l'invention.

Selon l'invention, le support est une alumine c'est-à-dire que le support comporte au moins 95%, de préférence au moins 98%, et de manière particulièrement préférée au moins 99% poids d'alumine par rapport au poids du support. L'alumine présente généralement une structure cristallographique du type alumine delta, gamma ou thêta, seule ou en mélange. Selon l'invention, le support d'alumine, peut comprendre des impuretés telles que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium, ou encore des métaux alcalins, de préférence le lithium, le sodium ou le potassium, et/ou les alcalino-terreux, de préférence le magnésium, le calcium, le strontium ou le baryum ou encore du soufre.

Avantageusement, la teneur en soufre du support d'alumine est comprise entre 0,001% et 2% poids par rapport au poids total du support d'alumine, et la teneur en sodium dudit support d'alumine est comprise entre 0,001% et 2% poids par rapport au poids total dudit gel d'alumine.

La surface spécifique de l'alumine est généralement comprise entre 10 m²/g et 250 m²/g, de préférence entre 30 m²/g et 200 m²/g, de façon plus préférée entre 50 m²/g et 150m²/g.

Le volume poreux de l'alumine est généralement compris entre 0,1 ml/g et 1,2 ml/g, de préférence compris entre 0,3 ml/g et 0,9 ml/g, et de manière très préférée compris entre 0,5 ml/g et 0,9 ml/g.

### Procédé de préparation du catalyseur

Un objet selon l'invention concerne un procédé de préparation d'un catalyseur selon l'invention comprenant au moins les étapes suivantes :
a) on approvisionne un gel d'alumine ayant avantageusement une teneur en soufre comprise entre 0,001 % et 2 % poids par rapport au poids total dudit gel d'alumine, et une teneur en sodium comprise entre 0,001 % et 2 % poids par rapport au poids total dudit gel d'alumine ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on met en contact le support d'alumine obtenu à l'issue de l'étape c) avec au moins un précurseur de la phase active de nickel pour obtenir un précurseur de catalyseur ;
e) on sèche le précurseur de catalyseur obtenu à l'issue de l'étape d), à une température inférieure à 250°C ;
   e1) optionnellement, on réalise un traitement thermique du précurseur de catalyseur séché obtenu à l'issue de l'étape e) à une température comprise entre 250 et 1000°C pour obtenir un précurseur de catalyseur calciné ;
f) on met en contact le précurseur de catalyseur séché obtenu à l'issue de l'étape e), éventuellement le précurseur de catalyseur calciné obtenu à l'issue de l'étape e1), avec au moins une solution contenant au moins un additif organique choisi parmi les aldéhydes renfermant de 1 à 14 atomes de carbone par molécule, les cétones ou polycétones renfermant de 3 à 18 atomes de carbone par molécule, les éthers ou les esters renfermant de 2 à 14 atomes de carbone par molécule, les alcools ou polyalcools renfermant de 1 à 14 atomes de carbone par molécule et les acides carboxyliques ou polyacides carboxyliques renfermant de 1 à 14 atomes de carbone par molécule, le ratio molaire entre l'additif organique et le nickel étant supérieur à 0,05 mol/mol ;
g) on réalise un traitement hydrothermal du précurseur de catalyseur obtenu à l'issue de l'étape f) à une température comprise entre 100 et 200°C pendant une durée comprise entre 30 minutes et 5 heures sous flux gazeux comprenant entre 5 et 650 grammes d'eau par kilogramme de gaz sec ;
h) optionnellement, on réalise une étape de séchage entre 50 et 200°C du précurseur de catalyseur obtenu à l'issue de l'étape g) sous flux gazeux comprenant une quantité d'eau inférieure strictement à 5 grammes d'eau par kilogramme de gaz sec.

L'ordre des étapes de a) à h) n'est pas permutable. Cependant, des étapes intermédiaires peuvent s'intercaler (notamment des étapes de séchages supplémentaires) et certaines étapes peuvent être effectuées plusieurs fois de suite (par exemple l'étape d)). Enfin, il est possible d'ajouter des étapes supplémentaires avant utilisation du catalyseur à l'issue de l'étape h).

De préférence, on réalise une étape de séchage puis une étape de calcination à l'issue de l'étape b) de mise en forme (mais avant la réalisation de l'étape c).

De préférence, les étapes les étapes e1) et h) ne sont pas optionnelles.

Les étapes a) à h) dudit procédé de préparation sont décrites en détail ci-après.

### Etape a)

Le catalyseur comprend un support alumine qui est obtenu à partir d'une alumine gel (ou gel d'alumine) qui comprend essentiellement un précurseur du type oxy(hydroxyde) d'aluminium (AIO(OH)) - également dénommé boehmite.

Selon l'invention, le gel d'alumine (ou autrement dénommé gel de boehmite) est synthétisé par précipitation de solutions basiques et/ou acides de sels d'aluminium induite par changement de pH ou tout autre méthode connue de la personne de métier (P. Euzen, P. Raybaud, X. Krokidis, H. Toulhoat, J.L. Le Loarer, J.P. Jolivet, C. Froidefond, Alumina, in Handbook of Porous Solids, Eds F. Schüth, K.S.W. Sing, J. Weitkamp, Wiley-VCH, Weinheim, Germany, 2002, pp. 1591-1677).

Généralement la réaction de précipitation est effectuée à une température comprise entre 5°C et 80°C et à un pH compris entre 6 et 10. De manière préférée la température est comprise entre 35°C et 70°C et le pH est compris entre 6 et 10.

Selon un mode de réalisation, l'alumine gel est obtenue par mise en contact d'une solution aqueuse d'un sel acide d'aluminium avec une solution basique. Par exemple le sel acide d'aluminium est choisi dans le groupe constitué par le sulfate d'aluminium, le nitrate d'aluminium ou le chlorure d'aluminium et de manière préférée, ledit sel acide est le sulfate d'aluminium. La solution basique est préférentiellement choisi parmi la soude ou la potasse. Alternativement, on peut mettre en contact une solution alcaline de sels d'aluminium qui peuvent être choisis dans le groupe constitué par l'aluminate de sodium et l'aluminate de potassium avec une solution acide. Dans une variante très préférée, le gel est obtenu par mise en contact d'une solution d'aluminate de sodium avec de l'acide nitrique. La solution d'aluminate de sodium présente avantageusement une concentration comprise entre 10⁻⁵ et 10⁻¹ mol.L⁻¹ et de manière préférée cette concentration est comprise entre 10⁻⁴ et 10⁻² mol.L⁻¹. Selon un autre mode de réalisation, l'alumine gel est obtenue par mise en contact d'une solution aqueuse de sels acides d'aluminium avec une solution alcaline de sels d'aluminium.

### Etape b)

Le support peut avantageusement être mis en forme par toute technique connue de la personne du métier. La mise en forme peut être réalisée par exemple par malaxage-extrusion, par pastillage, par la méthode de la coagulation en goutte (oil-drop), par granulation au plateau tournant ou par toute autre méthode bien connue de la personne du métier. Les catalyseurs préparés selon l'invention peuvent éventuellement être fabriqués et employés sous la forme d'extrudés, de tablettes, de billes. La méthode de mise en forme avantageuse selon l'invention est l'extrusion et les formes d'extrudés préférées sont cylindriques, cylindriques torsadées ou multilobées (2, 3, 4 ou 5 lobes par exemple).

Dans un mode de réalisation particulier, le gel d'alumine obtenu à l'issue de l'étape a) est soumis à une étape de malaxage de préférence dans un milieu acide. L'acide mis en oeuvre peut être par exemple de l'acide nitrique. Cette étape est réalisée au moyen d'outils connus tels que des malaxeurs bras en Z, des malaxeurs à meules, des mono ou bi-vis continues permettant la transformation du gel en un produit ayant la consistance d'une pâte. Selon un mode de réalisation avantageux, on apporte un ou plusieurs composés dits "agents porogènes" dans le milieu de malaxage. Ces composés présentent la propriété de se dégrader par chauffage et créer ainsi une porosité dans le support. Par exemple on peut utiliser comme composés porogènes la farine de bois, le charbon de bois, des goudrons, des matières plastiques. La pâte ainsi obtenue après malaxage est passée au travers d'une filière d'extrusion. Généralement les extrudés ont un diamètre compris 0,5 et 10 mm, de préférence entre 0,8 et 3,2 mm et de manière très préférée entre 1,0 et 2,5 mm et de longueur comprise entre 0,5 et 20 mm. Ces extrudés peuvent être de forme cylindrique, multilobée (par exemple trilobée ou quadrilobée).

Après sa mise en forme, le support est éventuellement séché avant de subir le traitement hydrothermal selon l'étape c) du procédé. Par exemple le séchage est effectué à une température comprise entre 50 et 200°C. Le support séché est éventuellement calciné avant de subir le traitement hydrothermal selon l'étape c) du procédé. Par exemple, la calcination est effectuée à une température comprise entre 200 et 1000°C, en présence ou non d'un flux d'air contenant jusqu'à 150 d'eau par kilogramme d'air sec.

### Etape c)

Le support obtenu à l'issue de l'étape b) subit ensuite une étape de traitement thermique qui permet de lui conférer des propriétés physiques répondant à l'application envisagée.

On désigne par le terme "traitement hydrothermal", un traitement par passage en autoclave en présence d'eau à une température supérieure à la température ambiante.

Au cours de ce traitement hydrothermal, on peut traiter de différentes manières l'alumine mise en forme. Ainsi, on peut imprégner l'alumine d'une solution acide, préalablement à son passage à l'autoclave, le traitement hydrothermal de l'alumine pouvant être fait soit en phase vapeur, soit en phase liquide, cette phase vapeur ou liquide de l'autoclave pouvant être acide ou non. Cette imprégnation, avant le traitement hydrothermal, peut être effectuée à sec ou par immersion de l'alumine dans une solution aqueuse acide. Par imprégnation à sec, on entend une mise en contact de l'alumine avec un volume de solution inférieur ou égal au volume poreux total de l'alumine traitée. De préférence, l'imprégnation est réalisée à sec. On peut également traiter le support extrudé sans imprégnation préalable par une solution acide, l'acidité étant dans ce cas apportée par le liquide aqueux de l'autoclave.

La solution aqueuse acide comprend au moins un composé acide permettant de dissoudre au moins une partie de l'alumine des extrudés. On entend par "composé acide permettant de dissoudre au moins une partie de l'alumine des extrudés", tout composé acide qui, mis en contact avec les extrudés d'alumine, réalise la mise en solution d'au moins une partie des ions aluminium. L'acide doit, de préférence, dissoudre au moins 0,5 % en poids d'alumine des extrudés d'alumine.

De préférence, cet acide est choisi parmi les acides forts tels que l'acide nitrique, l'acide chlorhydrique, l'acide perchlorique, l'acide sulfurique ou un acide faible mis en oeuvre à une concentration telle que sa solution aqueuse présente un pH inférieur à 4, tel que l'acide acétique, ou un mélange de ces acides.

Selon un mode préféré, on réalise le traitement hydrothermal en présence d'acide nitrique et d'acide acétique pris seul ou en mélange. L'autoclave est de préférence un autoclave à panier rotatif tel que celui défini dans la demande de brevet EP-A-0 387 109.

Le traitement hydrothermal peut également être réalisé sous pression de vapeur saturante ou sous une pression partielle de vapeur d'eau au moins égale à 70 % de la pression de vapeur saturante correspondant à la température de traitement.

De préférence le traitement hydrothermal est conduit à une température comprise entre 100 et 800°C, de préférence entre 200 et 700°C. Le traitement hydrothermal est généralement effectué entre 30 minutes et 8 heures, de préférence entre 30 minutes et 3 heures.

De préférence, l'étape de calcination qui a lieu après le traitement hydrothermal par passage en autoclave se déroule à une température généralement comprise entre 400 et 1500°C, de préférence entre 800 et 1300°C, généralement pendant 1 et 5 heures, sous air dont la teneur en eau est généralement comprise entre 0 et 700 g d'eau par kilogramme d'air sec.

A l'issue de l'étape c), l'alumine obtenue présente les propriétés texturales spécifiques telles que décrites ci-avant.

### Etape d)

La mise en contact du support avec une solution contenant un précurseur de nickel, conformément à la mise en oeuvre de l'étape d), peut être réalisée par imprégnation, à sec ou en excès, ou encore par dépôt - précipitation, selon des méthodes bien connues de la personne du métier.

Ladite étape d) est préférentiellement réalisée par imprégnation du support consistant par exemple en la mise en contact du support avec au moins une solution aqueuse contenant un précurseur de nickel. Le pH de ladite solution pourra être modifié par l'ajout éventuel d'un acide ou d'une base.

De manière préférée, ladite étape d) est réalisée par imprégnation à sec, laquelle consiste à mettre en contact le support avec au moins une solution, contenant, de préférence constituée de, au moins un précurseur du nickel, dont le volume de la solution est compris entre 0,25 et 1,5 fois le volume poreux du support à imprégner.

De manière préférée, ledit précurseur de nickel est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. De manière préférée, on utilise avantageusement comme précurseur de nickel, le nitrate de nickel, le chlorure de nickel, l'acétate de nickel ou le hydroxycarbonate de nickel. De manière très préférée, le précurseur de nickel est le nitrate de nickel.

Selon une autre variante, la solution aqueuse peut contenir de l'ammoniaque ou des ions ammonium NH₄⁺.

La concentration en nickel en solution est ajustée selon le type imprégnation (imprégnation à sec ou en excès) et le volume poreux du support de façon à obtenir pour le catalyseur supporté, une teneur en nickel comprise entre 1 et 50 % poids en élément nickel par rapport au poids total du catalyseur, plus préférentiellement entre 2 et 40 % poids et encore plus préférentiellement entre 3 et 35 % poids et encore plus préférentiellement 5 et 25 % poids.

### Etape e)

L'étape de séchage est effectuée sous flux gazeux comprenant une quantité d'eau inférieure à 150 grammes d'eau par kilogramme de gaz sec, de préférence inférieure à 50 g d'eau par kilogramme de gaz sec, à une température inférieure à 250°C, de préférence comprise entre 15 et 240°C, plus préférentiellement entre 30 et 220°C, encore plus préférentiellement entre 50 et 200°C, et de manière encore plus préférentielle entre 70 et 180°C, généralement pendant une durée comprise entre 10 minutes et 24 heures. Des durées plus longues ne sont pas exclues, mais n'apportent pas nécessairement d'amélioration.

Le gaz peut contenir de l'oxygène, de l'azote ou un gaz inerte et de préférence le gaz est l'air.

### Etape e 1) optionnelle

L'étape de calcination optionnelle est effectuée sous flux gazeux comprenant une quantité d'eau inférieure à 150 grammes d'eau par kilogramme de gaz sec, de préférence inférieure à 50 g d'eau par kilogramme de gaz sec, à une température comprise entre 250°C et 1000°C, de préférence entre 250 et 750°C. La durée de ce traitement thermique est généralement comprise entre 15 minutes et 10 heures. Des durées plus longues ne sont pas exclues, mais n'apportent pas nécessairement d'amélioration.

Le gaz peut contenir de l'oxygène, de l'azote ou un gaz inerte et de préférence le gaz est de l'air.

A l'issue des étapes e) ou e1), le nickel est réparti de façon homogène sur le support.

### Etape f)

Selon l'étape f) du procédé de préparation du catalyseur, on met en contact le précurseur de catalyseur obtenu à l'issue de l'étape e), éventuellement à l'issue de l'étape e1), avec au moins une solution comprenant au moins un additif organique choisi parmi les aldéhydes renfermant de 1 à 14 atomes de carbone par molécule (de préférence de 2 à 12), les cétones ou polycétones renfermant de 3 à 18 (de préférence de 3 à 12) atomes de carbone par molécule, les éthers ou les esters renfermant de 2 à 14 (de préférence de 3 à 12) atomes de carbone par molécule, les alcools ou polyalcools renfermant de 1 à 14 (de préférence de 2 à 12) atomes de carbone par molécule et les acides carboxyliques ou polyacides carboxyliques renfermant de 1 à 14 (de préférence de 1 à 12) atomes de carbone par molécule. L'additif organique peut être composé d'une combinaison des différents groupes fonctionnels cités ci-dessus.

De préférence, l'additif organique est choisi parmi l'acide formique HCOOH, le formaldéhyde CH₂O, l'acide acétique CH₃COOH, l'acide citrique, l'acide oxalique, l'acide glycolique (HOOC-CH₂-OH), l'acide malonique (HOOC-CH₂-COOH), l'éthanol, le méthanol, le formiate d'éthyle HCOOC₂H₅, le formiate de méthyle HCOOCH₃, le paraldéhyde (CH₃-CHO)₃, l'acétaldéhyde C₂H₄O, l'acide gamma-valérolactone (C₅H₈O₂), le glucose et le sorbitol, le trioxane.

De manière particulièrement préférée, l'additif organique est l'acide formique.

Il est essentiel que l'étape d'ajout de l'additif organique sur le catalyseur (étape f)) soit réalisée après l'étape de mise en contact du support avec le précurseur de la phase active de nickel.

De manière préférée, ladite étape f) est réalisée par imprégnation du précurseur de catalyseur obtenu à l'issue de la mise en oeuvre de l'étape e) ou de l'étape e1), avec une solution comprenant au moins un additif organique tel que cité ci-avant. L'imprégnation est généralement effectuée en solution aqueuse ou en solution organique ou en suspension dans la solution aqueuse ou organique, de préférence en solution aqueuse. Lorsque l'on opère en solution ou suspension organique, on utilisera à titre de solvant organique de préférence un alcool ou polyalcool, glycol ou polyglycol.

De manière préférée, ladite étape f) est réalisée par imprégnation à sec, laquelle consiste à mettre en contact le précurseur de catalyseur obtenu à l'issue de la mise en oeuvre de l'étape e) ou de l'étape e1), avec une solution comprenant au moins un additif organique tel que cité ci-avant, dont le volume de la solution est compris entre 0,25 et 1,5 fois le volume poreux du précurseur de catalyseur à imprégner.

L'imprégnation est généralement réalisée à une température entre 0 et 50°C, de préférence entre 10 et 40°C, et de manière particulièrement préférée à température ambiante.

Selon l'invention, le ratio molaire entre l'additif organique et le nickel est supérieur à 0,05 mol/mol, de préférence compris entre 0,1 et 5 mol/mol, plus préférentiellement compris entre 0,12 et 3 mol/mol, et de façon encore plus préférée compris entre 0,15 et 2,5 mol/mol.

### Etape g)

Selon l'étape g) du procédé de préparation du catalyseur selon l'invention, on effectue un traitement hydrothermal du produit issu de l'étape f) à une température comprise entre 100°C et 200°C, de préférence entre 130°C et 170°C, et plus particulièrement autour de 150°C, sous flux gazeux comprenant entre 5 et 650 grammes d'eau par kilogramme de gaz sec, de préférence entre 7 et 150 grammes d'eau par kilogramme de gaz sec, de façon encore plus préférée entre 10 et 50 grammes d'eau par kilogramme de gaz sec. Le gaz peut contenir de l'oxygène, de l'azote ou un gaz inerte et de préférence le gaz est de l'air.

La durée du traitement hydrothermal est généralement entre 30 minutes et 5 heures, de préférence entre 1 à 3 heures.

### Etape h) (optionnelle)

L'étape g) peut être suivie d'une étape h) de séchage entre 50 et 200°C sous flux gazeux comprenant une quantité d'eau inférieure strictement à 5 grammes d'eau par kilogramme de gaz sec, avantageusement pendant une durée comprise entre 30 minutes et 5 heures, de préférence entre 1 à 3 heures.

Le gaz peut contenir de l'oxygène, de l'azote ou un gaz inerte et de préférence le gaz est de l'air.

A l'issue de l'étape g) ou éventuellement de l'étape h), on obtient un catalyseur « semi eggshell » tel que représenté schématiquement en figure 1 et dont les caractéristiques sont décrites ci-dessus.

### Etape i) (optionnelle)

Préalablement à l'utilisation du catalyseur dans le réacteur catalytique et la mise en oeuvre d'un procédé d'hydrogénation, on effectue avantageusement au moins une étape de traitement réducteur i) en présence d'un gaz réducteur après les étapes g) ou h) de manière à obtenir un catalyseur comprenant du nickel au moins partiellement sous forme métallique.

Ce traitement permet d'activer ledit catalyseur et de former des particules métalliques, en particulier du nickel à l'état zéro valent. Ledit traitement réducteur peut être réalisé *in-situ* ou *ex-situ* c'est-à-dire après ou avant le chargement du catalyseur dans le réacteur d'hydrogénation.

Le gaz réducteur est de préférence l'hydrogène. L'hydrogène peut être utilisé pur ou en mélange (par exemple un mélange hydrogène / azote, ou hydrogène / argon, ou hydrogène / méthane). Dans le cas où l'hydrogène est utilisé en mélange, toutes les proportions sont envisageables.

Ledit traitement réducteur est réalisé à une température comprise entre 120 et 500°C, de préférence entre 150 et 450°C. Lorsque le catalyseur ne subit pas de passivation, ou subit un traitement réducteur avant passivation, le traitement réducteur est effectué à une température comprise entre 180 et 500°C, de préférence entre 200 et 450°C, et encore plus préférentiellement entre 350 et 450°C. Lorsque le catalyseur a subi au préalable une passivation, le traitement réducteur est généralement effectué à une température comprise entre 120 et 350°C, de préférence entre 150 et 350°C.

La durée du traitement réducteur est généralement comprise entre 2 et 40 heures, de préférence entre 3 et 30 heures. La montée en température jusqu'à la température de réduction désirée est généralement lente, par exemple fixée entre 0,1 et 10°C/min, de préférence entre 0,3 et 7°C/min.

Le débit d'hydrogène, exprimé en L/heure/gramme de catalyseur est compris entre 0,01 et 100 L/heure/gramme de catalyseur, de préférence entre 0,05 et 10 L/heure/gramme de catalyseur, de façon encore plus préférée entre 0,1 et 5 L/heure/gramme de catalyseur.

### Procédé d'hydrogénation sélective

Le catalyseur préparé selon le procédé de l'invention peut être utilisé dans un procédé d'hydrogénation sélective de composés polyinsaturés contenant au moins 2 atomes de carbone par molécule, tels que les dioléfines et/ou les acétyléniques et/ou les alcénylaromatiques, aussi appelés styréniques, contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur obtenu par le procédé de préparation tel que décrit ci-avant dans la description.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la coupe C5+ (composés hydrocarbonés ayant au moins 5 atomes de carbone), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 300°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C2-C3 de vapocraquage, une coupe C3 de vapocraquage, une coupe C4 de vapocraquage, une coupe C5 de vapocraquage et une essence de vapocraquage encore appelée essence de pyrolyse ou coupe C5+.

La coupe C2 de vapocraquage, avantageusement utilisée pour la mise en oeuvre du procédé d'hydrogénation sélective présente par exemple la composition suivante : entre 40 et 95 % poids d'éthylène, de l'ordre de 0,1 à 5 % poids d'acétylène, le reste étant essentiellement de l'éthane et du méthane. Dans certaines coupes C2 de vapocraquage, entre 0,1 et 1 % poids de composés en C3 peut aussi être présent.

La coupe C3 de vapocraquage, avantageusement utilisée pour la mise en oeuvre du procédé d'hydrogénation sélective présente par exemple la composition moyenne suivante : de l'ordre de 90 % poids de propylène, de l'ordre de 1 à 8 % poids de propadiène et de méthylacétylène, le reste étant essentiellement du propane. Dans certaines coupes C3, entre 0,1 et 2 % poids de composés en C2 et de composés en C4 peut aussi être présent.

Une coupe C2 - C3 peut aussi être avantageusement utilisée pour la mise en oeuvre du procédé d'hydrogénation sélective Elle présente par exemple la composition suivante : de l'ordre de 0,1 à 5 % poids d'acétylène, de l'ordre de 0,1 à 3 % poids de propadiène et de méthylacétylène, de l'ordre de 30 % poids d'éthylène, de l'ordre de 5 % poids de propylène, le reste étant essentiellement du méthane, de l'éthane et du propane. Cette charge peut aussi contenir entre 0,1 et 2 % poids de composés en C4.

La coupe C4 de vapocraquage, avantageusement utilisée pour la mise en oeuvre du procédé d'hydrogénation sélective présente par exemple la composition massique moyenne suivante : 1 % poids de butane, 46,5 % poids de butène, 51 % poids de butadiène, 1,3 % poids de vinylacétylène et 0,2 % poids de butyne. Dans certaines coupes C4, entre 0,1 et 2 % poids de composés en C3 et de composés en C5 peut aussi être présent.

La coupe C5 de vapocraquage, avantageusement utilisée pour la mise en oeuvre du procédé d'hydrogénation sélective présente par exemple la composition suivante : 21 % poids de pentanes, 45 % poids de pentènes, 34 % poids de pentadiènes.

L'essence de vapocraquage ou essence de pyrolyse, avantageusement utilisée pour la mise en oeuvre du procédé d'hydrogénation sélective correspond à une coupe hydrocarbonée dont la température d'ébullition est généralement comprise entre 0 et 300°C, de préférence entre 10 et 250°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alphaméthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante: 5 à 30 % poids de composés saturés (paraffines et naphtènes), 40 à 80 % poids de composés aromatiques, 5 à 20 % poids de mono-oléfines, 5 à 40 % poids de dioléfines, 1 à 20 % poids de composés alcénylaromatiques, l'ensemble des composés formant 100 %. Elle contient également de 0 à 1000 ppm poids de soufre, de préférence de 0 à 500 ppm poids de soufre.

De manière préférée, la charge d'hydrocarbures polyinsaturés traitée conformément au procédé d'hydrogénation sélective est une coupe C2 de vapocraquage, ou une coupe C2-C3 de vapocraquage, ou une essence de vapocraquage.

Le procédé d'hydrogénation sélective vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en oeuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs réinjection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en oeuvre technologique du procédé d'hydrogénation sélective peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation sélective des coupes C2, C2-C3, C3, C4, C5 et C5+ de vapocraquage peut être réalisée en phase gazeuse ou en phase liquide, de préférence en phase liquide pour les coupes C3, C4, C5 et C5+ et en phase gazeuse pour les coupes C2 et C2-C3. Une réaction en phase liquide permet d'abaisser le coût énergétique et d'augmenter la durée de cycle du catalyseur.

D'une manière générale, l'hydrogénation sélective d'une charge d'hydrocarbures contenant des composés polyinsaturés contenant au moins 2 atomes de carbone par molécule et ayant un point d'ébullition final inférieur ou égal à 300°C s'effectue à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire (définie comme le rapport du débit volumique de charge sur le volume du catalyseur) comprise entre 0,1 et 200 h⁻¹ pour un procédé réalisé en phase liquide, ou à un ratio molaire hydrogène/(composés polyinsaturés à hydrogéner) compris entre 0,5 et 1000 et à une vitesse volumique horaire comprise entre 100 et 40000 h⁻¹ pour un procédé réalisé en phase gazeuse.

Dans un mode de réalisation lorsqu'on effectue un procédé d'hydrogénatior sélective dans lequel la charge est une essence de vapocraquage comportant des composés polyinsaturés, le ratio molaire (hydrogène)/(composés polyinsaturés à hydrogéner) est généralement compris entre 0,5 et 10, de préférence entre 0,7 et 5,0 et de manière encore plus préférée entre 1,0 et 2,0, la température est comprise entre 0 et 200°C, de préférence entre 20 et 200 °C et de manière encore plus préférée entre 30 et 180°C, la vitesse volumique horaire (V.V.H.) est comprise généralement entre 0,5 et 100 h⁻¹, de préférence entre 1 et 50 h⁻¹ et la pression est généralement comprise entre 0,3 et 8,0 MPa, de préférence entre 1,0 et 7,0 MPa et de manière encore plus préférée entre 1,5 et 4,0 MPa. Plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 0,7 et 5,0, la température est comprise entre 20 et 200 °C, la vitesse volumique horaire (V.V.H.) est comprise généralement entre 1 et 50 h⁻¹ et la pression est comprise entre 1,0 et 7,0 MPa. Encore plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 1,0 et 2,0, la température est comprise entre 30 et 180°C, la vitesse volumique horaire (V.V.H.) est comprise généralement entre 1 et 50 h⁻¹ et la pression est comprise entre 1,5 et 4,0 MPa. Le débit d'hydrogène est ajusté afin d'en disposer en quantité suffisante pour hydrogéner théoriquement l'ensemble des composés polyinsaturés et de maintenir un excès d'hydrogène en sortie de réacteur.

Dans un autre mode de réalisation lorsqu'on effectue un procédé d'hydrogénation sélective dans lequel la charge est une coupe C2 de vapocraquage et/ou une coupe C2-C3 de vapocraquage comportant des composés polyinsaturés, le ratio molaire (hydrogène)/(composés polyinsaturés à hydrogéner) est généralement compris entre 0,5 et 1000, de préférence entre 0,7 et 800, la température est comprise entre 0 et 300°C, de préférence entre 15 et 280 °C, la vitesse volumique horaire (V.V.H.) est comprise généralement entre 100 et 40000 h⁻¹, de préférence entre 500 et 30000 h⁻¹ et la pression est généralement comprise entre 0,1 et 6,0 MPa, de préférence entre 0,2 et 5,0 MPa.

### Procédé d'hydrogénation des aromatiques

Le catalyseur préparé selon le procédé de l'invention peut être utilisé dans un procédé d'hydrogénation d'au moins un composé aromatique ou polyaromatique contenu dans une charge d'hydrocarbures ayant un point d'ébullition final inférieur ou égal à 650°C, généralement entre 20 et 650°C, et de préférence entre 20 et 450°C. Ladite charge d'hydrocarbures contenant au moins un composé aromatique ou polyaromatique peut être choisi parmi les coupes pétrolières ou pétrochimiques suivantes : le reformat du reformage catalytique, le kérosène, le gazole léger, le gazole lourd, les distillats de craquage, tels que l'huile de recyclage de FCC, le gazole d'unité de cokéfaction, les distillats d'hydrocraquage.

La teneur en composés aromatiques ou polyaromatiques contenus dans la charge d'hydrocarbures traitée dans le procédé d'hydrogénation est généralement compris entre 0,1 et 80% en poids, de préférence entre 1 et 50% en poids, et de manière particulièrement préférée entre 2 et 35% en poids, le pourcentage étant basé sur le poids total de la charge d'hydrocarbures. Les composés aromatiques présents dans ladite charge d'hydrocarbures sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou de chlore, de préférence inférieure à 100 ppm poids, et de manière particulièrement préférée inférieure à 10 ppm poids.

La mise en oeuvre technologique du procédé d'hydrogénation des composés aromatiques ou polyaromatiques est par exemple réalisée par injection, en courant ascendant ou descendant, de la charge d'hydrocarbures et de l'hydrogène dans au moins un réacteur à lit fixe. Ledit réacteur peut être de type isotherme ou de type adiabatique. Un réacteur adiabatique est préféré. La charge d'hydrocarbures peut avantageusement être diluée par une ou plusieurs ré-injection(s) de l'effluent, issu dudit réacteur où se produit la réaction d'hydrogénation des aromatiques, en divers points du réacteur, situés entre l'entrée et la sortie du réacteur afin de limiter le gradient de température dans le réacteur. La mise en oeuvre technologique du procédé d'hydrogénation des aromatiques selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation des composés aromatiques ou polyaromatiques peut être réalisée en phase gazeuse ou en phase liquide, de préférence en phase liquide. D'une manière générale, l'hydrogénation des composés aromatiques ou polyaromatiques s'effectue à une température comprise entre 30 et 350°C, de préférence entre 50 et 325°C, à une pression comprise entre 0,1 et 20 MPa, de préférence entre 0,5 et 10 MPa, à un ratio molaire hydrogène/(composés aromatiques à hydrogéner) entre 0,1 et 10 et à une vitesse volumique horaire comprise entre 0,05 et 50 h⁻¹, de préférence entre 0,1 et 10 h⁻¹ d'une charge d'hydrocarbures contenant des composés aromatiques ou polyaromatiques et ayant un point d'ébullition final inférieur ou égal à 650°C, généralement entre 20 et 650°C, et de préférence entre 20 et 450°C.

Le débit d'hydrogène est ajusté afin d'en disposer en quantité suffisante pour hydrogéner théoriquement l'ensemble des composés aromatiques et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière du procédé on réalise un procédé d'hydrogénation du benzène d'une charge d'hydrocarbures, tel que le reformat issu d'une unité de reformage catalytique. La teneur en benzène dans ladite charge d'hydrocarbures est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation du benzène contenu dans la charge d'hydrocarbures peut être réalisée en phase gazeuse ou en phase liquide, de préférence en phase liquide. Lorsqu'elle est réalisée en phase liquide, un solvant peut être présent, tel que le cyclohexane, l'heptane, l'octane. D'une manière générale, l'hydrogénation du benzène s'effectue à une température comprise entre 30 et 250°C, de préférence entre 50 et 200°C, et de manière plus préférée entre 80 et 180°C, à une pression comprise entre 0,1 et 10 MPa, de préférence entre 0,5 et 4 MPa, à un ratio molaire hydrogène/(benzène) entre 0,1 et 10 et à une vitesse volumique horaire comprise entre 0,05 et 50 h⁻¹, de préférence entre 0,5 et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention va maintenant être illustré via les exemples ci-après qui ne sont nullement limitatifs.

### Exemples

### Exemple 1 : Préparation de l'alumine AL-1

Un gel d'alumine est synthétisé via un mélange d'aluminate de sodium et de sulfate d'aluminium. La réaction de précipitation se fait à une température de 60°C, à un pH de 9, durant 60 minutes et sous une agitation de 200 tr/min.

Le gel ainsi obtenu subit un malaxage sur un malaxeur bras en Z pour fournir la pâte. L'extrusion est réalisée par passage de la pâte à travers une filière munie d'orifices de diamètre 1,6 mm en forme de trilobe. Les extrudés ainsi obtenus sont séchés à 150°C pendant 12 heures puis calcinés à 450°C sous flux d'air sec pendant 5 heures. L'air sec utilisé dans cet exemple et dans tous les exemples ci-dessous contient moins de 5 grammes d'eau par kilogramme d'air sec.

L'extrudé subit un traitement hydrothermal à 650°C en présence d'une solution aqueuse contenant de acide acétique à 6,5% poids par rapport au poids d"alumine pendant 3 heures en autoclave, puis est calciné sous flux d'air sec à 1000°C pendant 2 heures en réacteur tubulaire. On obtient l'alumine AL-1.

L'alumine AL-1 présente une surface spécifique de 80 m²/g, un volume poreux (déterminé par porosimétrie au Hg) de 0,85 mL/g et un diamètre médian mésoporeux de 35 nm. La teneur en sodium est de 0,0350%pds et la teneur en soufre de 0,15%pds.

### Exemple 1 bis : Préparation de l'alumine AL-2

Un gel d'alumine est synthétisé via un mélange d'aluminate de sodium et de sulfate d'aluminium. La réaction de précipitation se fait à une température de 60°C, à un pH de 9, durant 60 minutes et sous une agitation de 200 tr/min.

Le gel ainsi obtenu subit un malaxage sur un malaxeur bras en Z pour fournir la pâte. L'extrusion est réalisée par passage de la pâte à travers une filière munie d'orifices de diamètre 1,6 mm en forme de trilobe. Les extrudés ainsi obtenus sont séchés à 150°C pendant 12 heures puis calcinés à 450°C sous flux d'air sec pendant 5 heures.

On obtient l'alumine AL-2. Cette alumine ne subit pas de traitement hydrothermal.

L'alumine AL-2 présente une surface spécifique de 255 m²/g, un volume poreux (déterminé par porosimétrie au Hg) de 0,7 mL/g et un diamètre médian mésoporeux de 12 nm.

La teneur en sodium est de 0,0350%pds et la teneur en soufre de 0,15%pds.

### Exemple 2 : Préparation d'une solution aqueuse de précurseurs de Ni

La solution aqueuse de précurseurs de Ni (solution S) utilisée pour la préparation des catalyseurs A, C, D, E et F est préparée en dissolvant 43,5 grammes (g) de nitrate de nickel (NiNOs, fournisseur Strem Chemicals^{®}) dans un volume de 13 mL d'eau distillée. On obtient la solution S dont la concentration en Ni est de 350 g de Ni par litre de solution.

### Exemple 2 bis: Préparation d'une deuxième solution aqueuse de précurseurs de Ni

La solution aqueuse de précurseurs de Ni (solution S') utilisée pour la préparation du catalyseur B est préparée en dissolvant 14,5 grammes (g) de nitrate de nickel (NiNOs, fournisseur Strem Chemicals^{®}) dans un volume de 13 mL d'eau distillée. On obtient la solution S' dont la concentration en Ni est d'environ 116 g de Ni par litre de solution.

### Exemple 3 : Préparation d'un catalyseur A selon invention [15% poids de Ni - additif organique : acide formique]

La solution S préparée à l'exemple 2 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide formique avec un ratio molaire HCOOH/Ni égal à 1 mol/mol.

A l'issue de l'imprégnation de la solution aqueuse contenant l'acide formique, le précurseur de catalyseur subit un traitement thermique à 150°C, pendant 2 heures sous un flux d'air contenant 50 grammes d'eau par kilogramme d'air sec avec un débit de 1 L/h/g de catalyseur, puis pendant 1 heure à 120°C sous flux d'air sec.

On obtient le catalyseur A contenant 15 % en poids de l'élément nickel par rapport au poids total du catalyseur. Les caractéristiques du catalyseur A ainsi obtenu sont reportées dans le tableau 1 ci-après.

### Exemple 4 : Préparation d'un catalyseur B selon invention [5% poids de Ni - additif organique : acide formique]

La solution S' préparée à l'exemple 2 bis est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide formique, avec un ratio molaire HCOOH/Ni égal à 1 mol/mol. A l'issue de l'imprégnation de la solution aqueuse contenant l'acide formique, le précurseur de catalyseur subit un traitement thermique à 150°C pendant 2 heures sous un flux d'air contenant 50 grammes d'eau par kilogramme d'air sec avec un débit de 1 L/h/g de catalyseur, puis pendant 1 heure à 120°C sous flux d'air sec.

On obtient le catalyseur B contenant 5 % en poids de l'élément nickel par rapport au poids total du catalyseur. Les caractéristiques du catalyseur B ainsi obtenu sont reportées dans le tableau 1 ci-dessous.

### Exemple 5 : Préparation d'un catalyseur C selon invention [15% poids de Ni - additif organique : acide glycolique]

La solution S préparée à l'exemple 2 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide glycolique, avec une ratio C₂H₄O₃/Ni égal à 2 mol/mol.

A l'issue de l'imprégnation de la solution aqueuse contenant l'acide glycolique, le précurseur de catalyseur subit un traitement thermique à 150°C pendant 2 heures sous un flux d'air contenant 50 grammes d'eau par kilogramme d'air sec avec un débit de 1 L/h/g de catalyseur, puis pendant 1 heure à 120°C sous flux d'air sec.

On obtient le catalyseur C contenant 15 % en poids de l'élément nickel par rapport au poids total du catalyseur. Les caractéristiques du catalyseur C ainsi obtenu sont reportées dans le tableau 1 ci-après.

### Exemple 6 : Préparation d'un catalyseur D non-conforme à l'invention [pas de traitement hydrothermal pour l'obtention du support d'alumine]

La solution S préparée à l'exemple 2 est imprégnée à sec, en l'ajoutant goutte-à-goutte, sur 10 g d'alumine AL-2 obtenue selon l'exemple 1 bis.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide formique, avec un ratio HCOOH/Ni égal à 1 mol/mol.

A l'issue de l'imprégnation de la solution aqueuse contenant l'acide formique, le précurseur de catalyseur subit un traitement thermique à 150°C pendant 2 heures sous un flux d'air contenant 50 grammes d'eau par kilogramme d'air sec avec un débit de 1 L/h/g de catalyseur, puis pendant 1 heure à 120°C sous flux d'air sec.

On obtient le catalyseur D contenant 15 % en poids de l'élément nickel par rapport au poids total du catalyseur. Les caractéristiques du catalyseur D ainsi obtenu sont reportées dans le tableau 1 ci-après.

### Exemple 7 : Préparation d'un catalyseur E non-conforme [pas d'additif organique, pas de traitement hydrothermal final]

La solution S préparée à l'exemple 2 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

On obtient alors le catalyseur E contenant 15 % en poids de l'élément nickel par rapport au poids total du catalyseur. Les caractéristiques du catalyseur E ainsi obtenu sont reportées dans le tableau 1 ci-après.

### Exemple 8 : Préparation d'un catalyseur F non-conforme [pas d'additif organique]

La solution S préparée à l'exemple 2 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

Le solide ainsi obtenu subit ensuite un traitement thermique à 150°C pendant 2 heures sous un flux d'air contenant 50 grammes d'eau par kilogramme d'air sec avec un débit de 1 L/h/g de catalyseur, puis pendant 1 heure à 120°C sous flux d'air sec.

On obtient alors le catalyseur F contenant 15 % en poids de l'élément nickel par rapport au poids total du catalyseur. Les caractéristiques du catalyseur F ainsi obtenu sont reportées dans le tableau 1 ci-après.

**Tableau 1 : Caractéristiques des catalyseurs A à F**

| Catalyseur | Support | Additif organique | Traitement hydro-thermal | Ni (% pds) | Taille des particules (nm) | Epaisseur de croûte / diamètre du grain (%) | d_{croute}/ d_{coeur} | Teneur en Ni dans la croûte / Ni total (%) |
|---|---|---|---|---|---|---|---|---|
| A (conforme) | AL-1 | Acide formique | oui | 15 | 14,1 | 6,8 | 5 | 66 |
| B (conforme) | AL-1 | Acide formique | oui | 5 | 8 | 3,8 | 12 | 71 |
| C (conforme) | AL-1 | Acide glycolique | oui | 15 | 13,9 | 4,7 | 10 | 70 |
| D (non conforme) | AL-2 | Acide formique | oui | 15 | 10 | <1 | 1,5 | 7 |
| E (non conforme) | AL-1 | - | non | 15 | 13,7 | Répartition homogène | - | - |
| F (non conforme) | AL-1 | - | oui | 15 | 14,5 | Répartition homogène | - | - |

### Exemple 9 : Tests catalytiques : performances en hydrogénation sélective d'un mélange contenant du styrène et de l'isoprène (A_{HYD1})

Les catalyseurs A à F décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 % pds styrène (fournisseur Sigma Aldrich^{®}, pureté 99%), 8 % pds isoprène (fournisseur Sigma Aldrich^{®}, pureté 99%), 84 % pds n-heptane (solvant) (fournisseur VWR^{®}, pureté > 99% chromanorm HPLC). Cette charge contient également des composés soufrés en très faible teneur : 10 ppm pds de soufre introduits sous forme de pentanethiol (fournisseur Fluka^{®}, pureté > 97%) et 100 ppm pds de soufre introduits sous forme de thiophène (fournisseur Merck^{®}, pureté 99%). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Préalablement à son introduction dans l'autoclave, une quantité de 3 mL de catalyseur est réduite *ex situ* sous un flux d'hydrogène de 1 L/h/g de catalyseur, à 400 °C pendant 16 heures (rampe de montée en température de 1 °C/min), puis elle est transvasée dans l'autoclave, à l'abri de l'air. Après ajout de 214 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 30°C. Au temps t=0, environ 30 g d'un mélange contenant du styrène, de l'isoprène, du n-heptane, du pentanethiol et du thiophène sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à F sont reportées dans le tableau 2 ci-après. Elles sont rapportées à l'activité catalytique (A_{HYD1}) mesurée pour le catalyseur D.

### Exemple 10 : Tests catalytiques : performances en hydrogénation du toluène (A_{HYD2})

Les catalyseurs A à F décrits dans les exemples ci-dessus sont également testés vis-à-vis de la réaction d'hydrogénation du toluène.

La réaction d'hydrogénation sélective est opérée dans le même autoclave que celui décrit à l'exemple 9.

Préalablement à son introduction dans l'autoclave, une quantité de 2 mL de catalyseur est réduite *ex situ* sous un flux d'hydrogène de 1 L/h/g de catalyseur, à 400°C pendant 16 heures (rampe de montée en température de 1°C/min), puis elle est transvasée dans l'autoclave, à l'abri de l'air. Après ajout de 216 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS^{®}, pureté > 99,8%) sont introduits dans l'autoclave (la composition initiale du mélange réactionnel est alors toluène 6 % pds / n-heptane 94 % pds) et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à F sont reportées dans le tableau 2 ci-après. Elles sont rapportées à l'activité catalytique (A_{HYD2}) mesurée pour le catalyseur D.

**Tableau 2 : Comparaison des performances des catalyseurs A à F en hydrogénation sélective d'un mélange contenant du styrène et de l'isoprène (A_{HYD1}) et en hydrogénation du toluène (A_{HYD2})**

| Catalyseur | Teneur en Ni° (% en poids) | A_{HYD1} (%) | A_{HYD2} (%) |
|---|---|---|---|
| A (conforme) | 15 | 175 | 160 |
| B (conforme) | 5 | 125 | 130 |
| C (conforme) | 15 | 180 | 175 |
| D (non conforme) | 15 | 100 | 100 |
| E (non conforme) | 15 | 77 | 75 |
| F (non conforme) | 15 | 60 | 57 |

Ceci montre bien les performances améliorées des catalyseurs A, B et C préparés selon l'invention. par rapport aux catalyseurs D, E et F non conformes.

Le catalyseur D est en retrait d'activité du fait de l'utilisation du support AL-2 dont la préparation ne suit pas le protocole décrit dans l'invention. Les catalyseurs E et F sont préparés sur un support aluminique selon l'invention mais pour le catalyseur E les étapes e) et f) n'ont pas été effectuées et pour le catalyseur F l'étape e) d'ajout de l'additif organique n'a pas été effectuée alors que l'étape f) a été effectuée. Dans ces deux cas, le nickel est réparti de façon homogène dans tout le grain de catalyseur. Les catalyseurs E et F ont dès lors une activité bien en retrait du catalyseur A en A_{HYD1} et A_{HYD2}. Ceci s'explique par la répartition du Ni en croûte sur les catalyseurs A, B, et C qui leur confèrent une activité nettement améliorée notamment dans les réactions rapides d'hydrogénation.

## Revendications

1. Procédé de préparation d'un catalyseur comprenant une phase active à base de nickel et un support d'alumine, ledit catalyseur comprenant entre 1 et 50 % poids de nickel élémentaire, mesurée par fluorescence X, par rapport au poids total du catalyseur, ledit catalyseur étant **caractérisé en ce que** :
- le nickel est réparti à la fois sur une croûte en périphérie du support, et à coeur du support, l'épaisseur de ladite croûte, mesurée par microsonde de Castaing, étant comprise entre 2% et 15% du diamètre du catalyseur ;
- le ratio de densité en nickel entre la croûte et le coeur est supérieur strictement à 3 ;
- ladite croûte comprend plus de 25% en poids d'élément nickel par rapport au poids total de nickel contenu dans le catalyseur,
- la taille des particules de nickel dans le catalyseur, mesurée sous forme oxyde, est comprise entre 7 et 25 nm., lequel procédé comprend les étapes suivantes :
a) on approvisionne un gel d'alumine ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on met en contact le support d'alumine obtenu à l'issue de l'étape c) avec au moins un précurseur de la phase active de nickel pour obtenir un précurseur de catalyseur,
e) on sèche le précurseur de catalyseur obtenu à l'issue de l'étape d) à une température inférieure à 250°C ;
f) on met en contact le précurseur de catalyseur séché obtenu à l'issue de l'étape e) avec au moins une solution contenant au moins un additif organique choisi parmi les aldéhydes renfermant 1 à 14 atomes de carbone par molécule, les cétones ou polycétones renfermant 3 à 18 atomes de carbone par molécule, les éthers et les esters renfermant 2 à 14 atomes de carbone par molécule, les alcools ou polyalcools renfermant 1 à 14 atomes de carbone par molécule et les acides carboxyliques ou polyacides carboxyliques renfermant 1 à 14 atomes de carbone par molécule, le ratio molaire entre l'additif organique et le nickel étant supérieur à 0,05 mol/mol ;
g) on réalise un traitement hydrothermal du précurseur de catalyseur obtenu à l'issue de l'étape f) à une température comprise entre 100 et 200°C sous flux gazeux comprenant entre 5 et 650 grammes d'eau par kg de gaz sec.

2. Procédé selon la revendication 1, lequel procédé comprenant en outre une étape h) de séchage du précurseur de catalyseur obtenu à l'issue de l'étape g) à une température comprise entre 50 et 200°C sous flux gazeux comprenant une quantité d'eau inférieure strictement à 5 grammes d'eau par kg de gaz sec.

3. Procédé selon l'une des revendications 1 ou 2, lequel procédé comprenant en outre une étape de calcination e1) du précurseur de catalyseur séché obtenu à l'issue de l'étape e), sous flux gazeux comprenant une quantité d'eau inférieure strictement à 150 grammes d'eau par kg de gaz sec à une température comprise entre 250°C et 1000°C.

4. Procédé selon l'une quelconque des revendications 1 à 3, dans lequel à l'étape f) l'additif organique est choisi parmi l'acide formique, le formaldéhyde, l'acide acétique, l'acide citrique, l'acide oxalique, l'acide glycolique, l'acide malonique, l'éthanol, le méthanol, le formiate d'éthyle, le formiate de méthyle, le paraldéhyde, l'acétaldéhyde, l'acide gamma-valérolactone, le glucose, le sorbitol et le trioxane.

5. Procédé selon l'une quelconque des revendications 1 à 4, dans lequel à l'étape f) l'additif organique est l'acide formique.

## Patentansprüche

1. Verfahren zur Herstellung eines Katalysators, der eine aktive Phase auf Nickelbasis und einen Aluminiumoxid-Träger umfasst, wobei der Katalysator zwischen 1 und 50 Gew.-% elementares Nickel, gemessen mittels Röntgenfluoreszenz, bezogen auf das Gesamtgewicht des Katalysators, umfasst, wobei der Katalysator **dadurch gekennzeichnet ist, dass**:
- das Nickel sowohl auf einer Hülle am Umfang des Trägers als auch im Kern des Trägers verteilt ist, wobei die mit einer Castaing-Mikrosonde gemessene Dicke der Hülle zwischen 2 % und 15 % des Durchmessers des Katalysators beträgt;
- das Verhältnis der Nickeldichte zwischen der Hülle und dem Kern streng größer als 3 ist;
- die Hülle mehr als 25 Gew.-% eines Nickelelements in Bezug auf das Gesamtgewicht des im Katalysator enthaltenen Nickels umfasst,
- die Größe der in Oxidform gemessenen Nickelpartikel im Katalysator zwischen 7 und 25 nm liegt, wobei das Verfahren die folgenden Schritte umfasst:
a) das Bereitstellen eines Aluminiumoxidgels;
b) das Formen des Aluminiumoxidgels von Schritt a);
c) das Aussetzen des nach Schritt b) erhaltenen geformten Aluminiumoxidgels gegenüber einer thermischen Behandlung, die mindestens einen Schritt einer hydrothermalen Behandlung in einem Autoklaven in Gegenwart einer sauren Lösung umfasst, bei einer Temperatur zwischen 100 und 800 °C, und mindestens einem Kalzinierungsschritt bei einer Temperatur zwischen 400 und 1500 °C, der nach dem Schritt der hydrothermalen Behandlung durchgeführt wird, wodurch ein Aluminiumoxid-Träger erhalten wird;
d) das In-Kontakt-Bringen des nach Schritt c) erhaltenen Aluminiumoxid-Trägers mit mindestens einer Vorstufe der aktiven Nickelphase, wodurch eine Katalysatorvorstufe erhalten wird,
e) das Trocknen der nach Schritt d) erhaltenen Katalysatorvorstufe bei einer Temperatur unter 250 °C;
f) das In-Kontakt-Bringen der nach Schritt e) erhaltenen getrockneten Katalysatorvorstufe mit mindestens einer Lösung, die mindestens ein organisches Additiv enthält, das ausgewählt ist aus Aldehyden, die 1 bis 14 Kohlenstoffatome pro Molekül umfassen, Ketonen oder Polyketonen, die 3 bis 18 Kohlenstoffatome pro Molekül umfassen, Ethern und Estern, die 2 bis 14 Kohlenstoffatome pro Molekül umfassen, Alkoholen oder Polyalkoholen, die 1 bis 14 Kohlenstoffatome pro Molekül umfassen, und Carbonsäuren oder Polycarbonsäuren, die 1 bis 14 Kohlenstoffatome pro Molekül umfassen, wobei das Stoffmengenverhältnis zwischen dem organischen Additiv und dem Nickel mehr als 0,05 mol/mol beträgt;
g) das Durchführen einer hydrothermalen Behandlung der nach Schritt f) erhaltenen Katalysatorvorstufe bei einer Temperatur zwischen 100 und 200 °C unter einem Gasstrom, der zwischen 5 und 650 g Wasser pro kg trockenem Gas umfasst.

2. Verfahren nach Anspruch 1, wobei das Verfahren weiterhin einen Schritt h) des Trocknens der nach Schritt g) erhaltenen Katalysatorvorstufe bei einer Temperatur zwischen 50 und 200 °C unter einem Gasstrom umfasst, der eine Wassermenge von streng weniger als 5 g Wasser pro kg trockenem Gases umfasst.

3. Verfahren nach einem der Ansprüche 1 oder 2, wobei das Verfahren weiterhin einen Schritt e1) des Kalzinierens der nach Schritt e) erhaltenen getrockneten Katalysatorvorstufe unter einem Gasstrom umfasst, der eine Wassermenge von streng weniger als 150 g Wasser pro kg trockenem Gas bei einer Temperatur zwischen 250 °C und 1000 °C umfasst.

4. Verfahren nach einem der Ansprüche 1 bis 3, wobei in Schritt f) das organische Additiv aus Ameisensäure, Formaldehyd, Essigsäure, Citronensäure, Oxalsäure, Glycolsäure, Malonsäure, Ethanol, Methanol, Ethylformiat, Methylformiat, Paraldehyd, Acetaldehyd, Gamma-Valerolactonsäure, Glucose, Sorbit und Trioxan ausgewählt ist.

5. Verfahren nach einem der Ansprüche 1 bis 4, wobei in Schritt f) das organische Additiv Ameisensäure ist.

## Claims

1. Process for preparing a catalyst comprising a nickel-based active phase and an alumina support, said catalyst comprising between 1% and 50% by weight of elemental nickel, measured by x-ray fluorescence, relative to the total weight of the catalyst, said catalyst being **characterized in that**:
- the nickel is distributed both on a crust at the periphery of the support, and in the core of the support, the thickness of said crust, measured by Castaing microprobe, being between 2% and 15% of the diameter of the catalyst;
- the nickel density ratio between the crust and the core is strictly greater than 3;
- said crust comprises more than 25% by weight of nickel element relative to the total weight of nickel contained in the catalyst,
- the size of the nickel particles in the catalyst, measured in oxide form, is between 7 and 25 nm, which process comprises the following steps:
a) an alumina gel is provided;
b) the alumina gel from step a) is shaped;
c) the shaped alumina gel obtained at the end of step b) is subjected to a heat treatment comprising at least one hydrothermal treatment step in an autoclave in the presence of an acid solution, at a temperature of between 100°C and 800°C, and at least one calcining step, at a temperature of between 400°C and 1500°C, carried out after the hydrothermal treatment step, in order to obtain an alumina support;
d) the alumina support obtained at the end of step c) is brought into contact with at least one precursor of the nickel active phase in order to obtain a catalyst precursor,
e) the catalyst precursor obtained at the end of step d) is dried at a temperature below 250°C;
f) the dried catalyst precursor obtained at the end of step e) is brought into contact with at least one solution containing at least one organic additive chosen from aldehydes containing 1 to 14 carbon atoms per molecule, ketones or polyketones containing 3 to 18 carbon atoms per molecule, ethers and esters containing 2 to 14 carbon atoms per molecule, alcohols or polyalcohols containing 1 to 14 carbon atoms per molecule and carboxylic acids or polycarboxylic acids containing 1 to 14 carbon atoms per molecule, the mole ratio between the organic additive and the nickel being greater than 0.05 mol/mol;
g) a hydrothermal treatment of the catalyst precursor obtained at the end of step f) is carried out at a temperature between 100°C and 200°C under a gas stream comprising between 5 and 650 grams of water per kg of dry gas.

2. Process according to Claim 1, which process further comprising a step h) of drying the catalyst precursor obtained at the end of step g) at a temperature between 50°C and 200°C under a gas stream comprising an amount of water strictly less than 5 grams of water per kg of dry gas.

3. Process according to either one of Claims 1 and 2, which process further comprising a step e1) of calcining the dried catalyst precursor obtained at the end of step e), under a gas stream comprising an amount of water strictly less than 150 grams of water per kg of dry gas at a temperature between 250°C and 1000°C.

4. Process according to any one of Claims 1 to 3, in which, in step f), the organic additive is chosen from formic acid, formaldehyde, acetic acid, citric acid, oxalic acid, glycolic acid, malonic acid, ethanol, methanol, ethyl formate, methyl formate, paraldehyde, acetaldehyde, gamma-valerolactone acid, glucose, sorbitol and trioxane.

5. Process according to any one of Claims 1 to 4, in which, in step f), the organic additive is formic acid.
